# EUROPEAN PATENT SPECIFICATION

(11) **EP 3 463 364 B1**
(45) Date of publication and mention of the grant of the patent: **09.10.2024**
(21) Application number: 17732994.3
(22) Date of filing: 23.05.2017
(51) Int. Cl.: A61K 31/497, A61P 35/00, A61P 43/00, A61P 31/12, A61P 3/00, A61P 9/10, A61P 9/00, A61P 7/00

(54) **PYRAZINE DERIVATIVES FOR USE IN THE TREATMENT OF DISEASES ASSOCIATED WITH CELL CYCLE DYSREGULATION**
PYRAZINDERIVATE ZUR VERWENDUNG IN DER BEHANDLUNG VON ERKRANKUNGEN IM ZUSAMMENHANG MIT DER ZELLZYKLUSDYSREGULATION
DÉRIVÉS DE PYRAZINE DESTINÉS POUR LEUR UTILISATION DANS LE TRAITEMENT DE MALADIES ASSOCIÉES À UN DÉRÈGLEMENT DU CYCLE CELLULAIRE

(30) Priority: 23.05.2016 GR 20160100281
(43) Date of publication of application: 10.04.2019
(73) Proprietor: University of Patras, 26504 Rio Achaia (GR); Taraviras, Stavros, 26504 Platani Achaias (GR); Lygeroy, Zoi, 26504 Platani Achaias (GR); Karantzelis, Nikolaos, 54453 Thessaloniki (GR)
(72) Inventor: TARAVIRAS, Stavros, 26504 Platani Achaias (GR); LYGEROY, Zoi, 26504 Platani Achaias (GR); KARANTZELIS, Nikolaos, 54453 Thessaloniki (GR)
(74) Representative: Dodou, Evdokia
(86) International application number: PCT/GR2017/000027
(87) International publication number: WO 2017/203304

(56) References cited:
- KATARZYNA GOBIS ET AL: "Studies on Pyrazine Derivatives, XLV: Synthesis, Reactions, and Tuberculostatic Activity of N -Methyl- N '-(pyrazine-2-carbonyl)-hydrazinecarbodithioic Acid Methyl Ester", PHOSPHORUS, SULFUR AND SILICON AND THE RELATED ELEMENTS, vol. 181, no. 5, 1 June 2006 (2006-06-01), US, pages 965 - 975, XP055355543, ISSN: 1042-6507, DOI: 10.1080/10426500500272319
- KATARZYNA GOBIS ET AL: "Studies on Pyrazine Derivatives, XLVI: The Synthesis of New Pyrazine Derivatives with N '-(Pyrazine-2-carbonyl)-hydrazinecarbodithioic Acid Methyl Ester Usage", PHOSPHORUS, SULFUR AND SILICON AND THE RELATED ELEMENTS, vol. 181, no. 5, 1 June 2006 (2006-06-01), US, pages 977 - 986, XP055355541, ISSN: 1042-6507, DOI: 10.1080/10426500500272327
- HENRYK FOKS ET AL: "STUDIES ON PYRAZINE DERIVATIVES. XXXI. SYNTHESIS AND REACTIONS OF ALKYL 3-PYRAZINOYLDITHIOCARBAZATES AND S,S'-DIALKYLDITHIOCARBONATE PYRAZINOYLHYDRAZONES TOWARDS AMINES AND HYDRAZINES", PHOSPHORUS, SULFUR AND SILICON AND THE RELATED ELEMENTS, vol. 158, no. 1, 1 March 2000 (2000-03-01), US, pages 107 - 116, XP055355547, ISSN: 1042-6507, DOI: 10.1080/10426500008042078

## Description

### Field of the invention

The present invention provides pyrazine derivatives for use in the treatment of human or animal diseases associated with cell cycle dysregulation, namely cancer. Also provided for use in the treatment of said disease are pharmaceutical compositions comprising the compounds of the present invention in combination with one or more pharmaceutically acceptable carriers, solvents or excipients.

### Technical background

Most of the now widely used anticancer drugs work by inducing replicative stress by damaging the integrity of DNA. The term replicative stress refers to structural damage of DNA and subsequent activation of the DNA damage response mechanism (DNA Damage Response, DDR). Some examples of such drugs are alkylating agents, platinum complexes and nucleotide analogs, which, despite their effectiveness, lack high specificity. Thus, it would be of great importance to develop an alternative way of inducing replicative stress that will be effective as well as of higher specificity.

One such way could be the dysregulation of the licensing system through the use of pharmacological molecules with targeted action against licensing agents. The licensing process of replication requires the stepwise assembly of a multiprotein complex, called pre-replicative complex (pre-RC) at replication origins. The proteins Geminin and Cdt1 play a key role in regulation of the licensing system and thus constitute promising targets for pharmacological interventions in diseases such as cancer, as well as in other diseases associated with cell cycle dysregulation.

Notably normal and cancer cells respond adversely to the disturbance of Geminin-Cdtl balance. It has been shown that inhibition of licensing through overexpression of Geminin leads to cell cycle arrest in normal cells whereas it induces apoptosis selectively in malignant cells. Furthermore, depletion of Geminin results in re-replication and apoptosis in cancer cells but not in normal cells. Regarding Cdtl dysregulation, it has been shown that overexpression of the protein causes genome over-replication and induces apoptosis in cancer cells, while in the case of normal cells it does not cause doubling of the genome although chromosomal abnormalities are observed.

In conclusion, the disturbance of Geminin-Cdtl balance can lead to a differential response between tumor and normal cells. The regulation of the balance and the activity of the two proteins is mainly dependent on their direct interaction. Thus, the protein complex Geminin-Cdtl constitutes an attractive and promising target for small molecule inhibitors of protein interactions that can affect the activity of these two proteins and be used as pharmacological molecules with selective antitumor activity.

So far there have been some studies on identifying molecules that inhibit the Geminin-Cdtl interaction. Specifically, Mizushina Y et al. have shown that coenzyme Q10 (Biochim Biophys Acta. 2008, Vol. 1780(2), pp 203-13) the glycolipid SQDG from spinach (Biochimie. 2008, Vol. 90(6) pp 947-56) and oleic acid (C18:1) (Int J Mol Med. 2008, Vol. 21(3), pp 281-90) inhibit the interaction of the two proteins in vitro, through their binding to the Cdtl licensing factor. Of note, the coenzyme Q10, the glycolipid SQDG from spinach and oleic acid, in contrast to synthetic chemical compounds, lack pharmacological properties because of their natural origin. This highlights the need to identify small molecule inhibitors of the complex Geminin-Cdtl that are amenable to pharmacological development and optimization.

Regarding the inhibitory effect of these molecules on the Geminin-Cdtl interaction, this is limited to inhibiting formation of the protein complex, as these molecules do not exhibit capacity to disrupt the complex. However, for an effective cancer treatment, identification of pharmacological molecules that have the ability to disrupt the Geminin-Cdtl protein complex is of vital importance. This is because in an asynchronous population of cancer cells - as it is usually the case in in vivo conditions - the two proteins will be in complex in a significant proportion of these cells.

Furthermore, the inhibitors Q10, SQDG and oleic acid have not been studied for their effect on cell cycle progression in cancer cells and normal cells. However, this is necessary in order to ascertain any anticancer activity and to determine their selectivity of action between cancer and normal cells.

Prior art relevant to certain of the compounds of the present invention is briefly discussed below:
Gobis K et al. (Phosphorus, Sulfur and Silicon. 2006, 181: 965-975) have reported the synthesis and tuberculostatic activity of new pyrazine derivatives using N-Methyl-N'-(pyrazine-2-carbonyl)-hydrazinecarbodithioic acid methyl ester.
Gobis K et al. (Phosphorus, Sulfur and Silicon. 2006, 181:977-986) have reported the synthesis and tuberculostatic activity of new pyrazine derivatives using N'-(pyrazine-2-carbonyl)-hydrazinecarbodithioic acid methyl ester.
Foks H. et al. (Phosphorus, Sulfur and Silicon. 2000, 158: 107-116) describes the synthesis of pyrazine compounds starting from methyl 3-pyrazinodithiocarbazate ester.

The problem solved by this invention relates to the need to develop novel pharmacologically active small molecules with targeted ability to induce replicative stress selectively in uncontrollably dividing cells, at least partly through a mechanism of disruption and/or inhibition of formation of the Geminin-Cdtl protein complex.

### Summary of the invention

The inventors have surprisingly found that the compounds of formula (I) possess selective ability to inhibit cell cycle progression of uncontrollably dividing cancer cells versus normally dividing cells. These characteristics make these compounds potentially useful in the treatment of human and mammalian diseases associated with dysregulation of the cell cycle. The compounds of formula (I) exhibit an ability to disrupt and/or inhibit formation of the Geminin-Cdtl complex *in vitro* as well as the ability to disrupt and/or inhibit formation of said complex in appropriate cellular assays. Also obtained herein are pharmaceutical compositions comprising one or more compounds of formula (I) or a salt thereof, in combination with one or more pharmaceutically acceptable carriers, solvents or excipients for use in the treatment of human and mammalian diseases associated with dysregulation of the cell cycle.

Diseases associated with dysregulation of the cell cycle comprise cancer, hamartoma, inflammatory disease, haematological disorder, viral disease with the potential of inducing carcinogenesis, metabolic disorder, progeroid syndrome, atherosclerosis, and vasculoproliferative disorder. According to the present invention, the disease associated with dysregulation of the cell cycle is cancer.

### Brief description of the drawings

The present invention will now be described with reference to certain embodiments thereof which are illustrated in the accompanying drawings. It should be noted that the accompanying drawings illustrate preferred embodiments of the invention therefore should not be considered as limiting the scope of the invention.
Fig. 1 shows the structure of the synthetic compound AF-615/30368009 (compound number 1 in Tables A and B).
Fig. 2 depicts the results of the *in vitro* study on the specificity of action and potency of the synthetic compound AF-615/30368009 using Surface Plasmon Resonance (SPR).
Fig. 3 shows the results of the *ex vivo* study of the specificity of action of the synthetic compound AF-615/30368009 through FRET Acceptor Photobleaching.
Fig. 4 shows the results of the *ex vivo* study of the mechanism of action of the synthetic compound AF-615/30368009 through its effect on the intracellular distribution of exogenous Geminin. The symbols «*» and «**» refer to p values ≤0.05 and ≤0.005, respectively.
Fig. 5 illustrates the effect of the synthetic compound AF-615/30368009 on cell cycle progression of cancer cells and normal cells, as seen with flow cytometry.

### Detailed Description of the Invention

According to the present invention, compounds of formula (I) are provided for use in the treatment of cancer wherein: 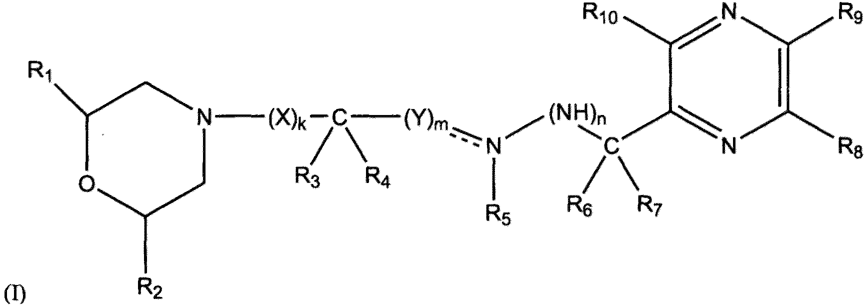
R₁ and R₂ are independently -H;
k is 0; R₃ and R₄ together form =S;
R₆ and R₇ together form =O;
m is 0;
R₅ is -H or CH₃;
n is 1;
R₈ is -H; R₉ is -H;
R₁₀ is -H;
or stereoisomers, enantiomers and/or pharmaceutically acceptable salts or hydrites thereof.

The present invention therefore describes compounds of formula (I) for use in the treatment of cancer, wherein said compounds are N'-(morpholine-4-carbothioyl)pyrazine-2-carbohydrazide, N'-methyl-N'-(morpholine-4-carbothioyl)pyrazine-2-carbohydrazide, or stereoisomers, enantiomers and/or pharmaceutically acceptable salts or hydrites thereof. Said compounds are listed in Table A.

**TABLE A**

| **No.** | **IUPAC NAME** |
|---|---|
| 1 | N'-(morpholine-4-carbothioyl)pyrazine-2-carbohydrazide |
| 2 | N'-methyl-N'-(morpholine-4-carbothioyl)pyrazine-2-carbohydrazide |

The correlation between formula (I) and the compounds listed in Table A based on the values of each of their substituents, is shown in Table B.

**TABLE B**

| **No.** | **R₁, R₂** | **k, m, n, X, Y** | **R₃, R₄** | **R₅** | **R₆, R₇** | **R₈, R₉, R₁₀** |
|---|---|---|---|---|---|---|
| 1 | R₁ : -H | k, m : 0 | =S | -H | =O | R₈ : -H |
| | R₂ : -H | n : 1 | | | | R₉ : -H |
| | | X, Y: Absent | | | | R₁₀ : -H |
| 2 | R₁ : -H | k, m: 0 | =S | -CH₃ | =O | R₈ : -H |
| | R₂ : -H | n : 1 | | | | R₉ : -H |
| | | X, Y: Absent | | | | R₁₀ : -H |

The term "acceptable salts" refers to pharmacologically acceptable salts that retain the desired biological activity of the compounds of the present invention and exhibit minimal or no adverse effects. The pharmacologically acceptable salts according to the invention include therapeutically active, non-toxic forms of acidic or basic salts, which the compounds of formula (I) are able to form.

Optically active carbon atoms present in the compounds of formula (I) can independently of each other be in the R or S configuration. The compounds of formula (I) may be present in the form of pure enantiomers or pure diastereomers or in the form of mixtures of enantiomers and/or diastereomers, for example in the form of racemic mixtures. The invention also includes mixtures of two or more stereoisomers of formula (I) and comprises all ratios of stereoisomers in the mixtures. Where the compounds of formula (I) may exist as E isomers or Z isomers (or cis isomers or trans isomers) the invention relates to both pure E isomers and pure Z isomers and to E/Z mixtures in all proportions. The invention also includes all tautomeric forms of the compounds of formula (I).

The compounds of formula (I) can be prepared using procedures and techniques which are well known and can be appreciated by one of ordinary technical experience. Precursors or building blocks for use in the general synthetic procedures that can be applied in the preparation of compounds of formula (I) are readily available to one of ordinary technical skill. In many cases, the compounds of formula (I) are commercially available or have been described in the literature. Diastereomers, including E/Z isomers, can be separated into the individual isomers, for example by chromatography. Racemic mixtures can be separated into the two enantiomers by customary methods, for example by chromatography on chiral phases or by separation, for example by crystallization of diastereomeric salts obtained with optically active acids or bases. Stereochemically uniform compounds of formula (I) may also be obtained by employing stereochemically uniform starting materials or by using stereoselective reactions.

The invention relates to the treatment of disease states associated with cell cycle dysregulation. These disease states are characterized by uncontrolled cell cycle re-entry of cells that had stopped dividing or by continuous cell divisions of one or more cells of a multicellular organism resulting in harm to the multicellular organism. The disease, syndrome or disorder to be treated with the compounds or pharmaceutical compositions described herein is cancer.

Non-limiting examples of cancers are brain tumors, e.g. glioblastoma and astrocytoma, cerebral metastases, cervical cancer, hepatocellular carcinoma, melanoma, prostate cancer, head and neck cancer, pancreatic cancer, bladder cancer, stomach cancer, renal cell carcinoma, ovarian cancer, sarcoma, and basal cell carcinoma.

The compounds of formula (I), pure enantiomers, pure diastereomers, racemic mixtures, salts or hydrates thereof, and prodrugs thereof may be administered to animals, preferably to mammals, and in particular to humans as pharmaceuticals for therapy or prophylaxis. They can be administered alone, or in combination with other active pharmaceuticals.

The active pharmaceutical ingredients that can be co-administered with the compounds of this invention include, among others, substances with anti-tumor action, immunosuppressive agents, anti-inflammatory substances, chemotherapeutic agents, pro-apoptotic agents, anti-viral agents, cholesterol lowering agents, anti-angiogenic factors, cell differentiation factors, or antioxidants. Examples of anticancer and chemotherapeutic agents are alkylating agents, platinum compounds, microtubule inhibitors, inhibitors of topoisomerase I and II, antimetabolites, antitumor antibiotics, kinase inhibitors, nucleoside analogues, aromatase inhibitors, anti-androgens, hormones and others. Examples of anti-inflammatory active compounds are cortisone, hydrocortisone, betamethasone, dexamethasone, prednisolone, methylprednisolone, prednisone, triamcinolone, salicylic acid, diclofenac, indomethacin, ibuprofen, piroxicam and flurbiprofen. Examples of anti-angiogenic agents are ranibizumab and bevacizumab. Examples of anti-viral agents are abacavir, acyclovir, amprenavir, brivudine, ribavirin and others.

In yet another embodiment of the present invention, provided are pharmaceutical compositions comprising a compound of formula (I) of the invention, a pure enantiomer, a pure diastereoisomer or a racemic mixture thereof, a salt or hydrate, and pharmaceutically acceptable auxiliary substances and excipients. These auxiliary substances that can be added to therapeutic agents are known to the expert and include preservatives, wetting or emulsifying agents, pH buffering agents, stabilizers, detergents, antioxidants, carriers, flavoring agents, phospholipids, fillers, disintegrants, binders, lubricants, wetting agents, stabilizers, sweeteners, colorants, thickening agents, solvents, solubilizers, agents for generating sustained release tablets, salts for varying the osmotic pressure, coating agents, and other factors.

The pharmacological form of the particular composition will generally depend on the method by which the composition is administered to a patient. In non-limiting embodiments, the pharmacological form is selected from the group comprising tablets, capsules, pills (gelcaps), soft capsules (softcaps) and suppositories. The pharmacological forms may have a solid form (including granules, powders or suppositories) or liquid (e.g., emulsions, syrups, injectable solutions, solutions for infusion).

The pharmaceuticals can be administered orally, for example in the form of pills, tablets, lacquered tablets, coated tablets, granules, hard and soft gelatine capsules, solutions, syrups, emulsions, suspensions or aerosol mixtures. Administration, however, can also be performed rectally, for example in the form of suppositories, or parenterally, for example intravenously, intramuscularly or subcutaneously, in the form of injection solutions or infusion solutions, microcapsules, implants, or transdermally, or locally, for example in the form of ointments, solutions or tinctures, or in other ways, for example in the form of aerosols or nasal sprays.

Formulations for oral administration in solid or liquid form can be prepared according to any method known in the manufacture of pharmaceutical compositions and such compositions may contain one or more agents selected from the group comprising sweetening agents, flavoring agents and colorants in order to provide palatable preparations. Other useful excipients may be for example, inert diluents such as calcium carbonate, sodium carbonate, lactose, calcium phosphate or sodium phosphate, granulating and disintegrating agents, as well as other non-toxic compatible fillers, binders, non-ionic, cationic and anionic surfactants, buffers, antioxidants, lubricants, preservatives or stabilizers, and the like commonly used in pharmaceutical formulations. The tablets may be uncoated or may be coated by known techniques. In some cases such coatings can be prepared by known techniques to delay disintegration and absorption in the gastrointestinal tract and thereby provide a sustained action over a longer period. For example, a time delay material may be used such as monoglycerides or diglycerides of stearic acid.

Suitable excipients for the production of solutions, for example injection solutions, or of emulsions or syrups are, for example, water, saline, alcohols, glycerol, polyols, sucrose, invert sugar, glucose, vegetable oils, and others. Suitable excipients for microcapsules or implants are, for example, copolymers of glycolic acid and lactic acid.

For rectal administration in the form of suppositories, the compositions can be prepared by mixing the drug with a suitable non-irritating excipient such as cocoa butter, synthetic glyceride esters or polyethylene glycols.

The pharmaceutical preparations normally contain about 0.5% to 90% by weight of the compounds of formula (1) and/or their pharmacologically acceptable salts and/or their prodrugs. The amount of the active ingredient of formula (I) and/or their pharmacologically acceptable salts in the pharmaceutical preparations normally is from about 0.5 mg to about 1000 mg, preferably from about 1 mg to about 500 mg.

The pharmaceutical preparations can also contain two or more compounds of formula (1), and/or their pharmacologically acceptable salts. Furthermore, apart from at least one compound of formula (1) and/or a pharmacologically acceptable thereof, the pharmaceutical preparations can also contain one or more therapeutically or prophylactically active ingredients. These active ingredients may be substances with anti-cancer activity, anti-inflammatory substances, chemotherapeutic agents, apoptotic agents, anti-viral agents, cholesterol lowering agents, antiangiogenic factors, cell differentiation factors or antioxidants, in therapeutically effective amounts.

Examples of anticancer and chemotherapeutic agents are alkylating agents, platinum compounds, microtubule inhibitors, inhibitors of topoisomerase I and II, antimetabolites, antitumor antibiotics, kinase inhibitors, nucleoside analogues, aromatase inhibitors, anti-androgens, hormones, and others. Examples of anti-inflammatory active compounds are cortisone, hydrocortisone, betamethasone, dexamethasone, prednisolone, methylprednisolone, prednisone, triamcinolone, salicylic acid, diclofenac, indomethacin, ibuprofen, piroxicam and flurbiprofen. Examples of anti-angiogenic agents are ranibizumab and bevacizumab. Examples of anti-viral agents are abacavir, acyclovir, amprenavir, brivudine, ribavirin, and others.

When using the compounds of formula (I) the dose can vary within wide limits and, as is customary and known to the physician, will be adapted to the specific conditions in each individual case. The dose depends, for example, on the specific compound employed, on the nature and severity of the disease to be treated, the manner and schedule of administration, or whether it is used for acute or chronic disease or as prophylaxis. The appropriate dosage can be established using clinical approaches well known in the medical art. In general, the daily dose for achieving the desired results in an adult weighing about 75 kg is from 0.01 mg/kg to 100 mg/kg, preferably from 0.1 mg/kg to 50 mg / kg, in particular from 0.1 mg/kg to 10 mg/kg (in each case in mg per kg of body weight). The daily dose can be divided, in particular in the case of administration of relatively large amounts, into several, for example 2, 3 or 4 individual administrations. Usually, depending on the response of the individual patient it may be necessary to adjust upwards or downwards the indicated daily dose.

### Examples

The following examples are given for purposes of illustrating various embodiments of the present invention and are not meant to limit the present invention in any fashion.

### Example 1

**In vitro control of the specificity and potency of action of the synthetic chemical compound AF-615/30368009 in inhibiting formation of the Geminin-Cdt1 protein complex**

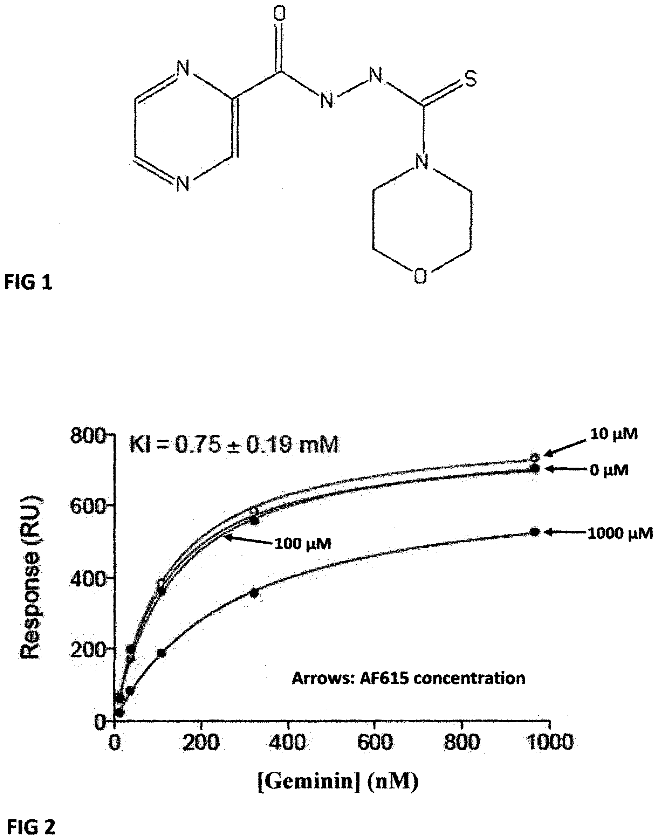

Surface plasmon resonance analysis was performed in order to determine in a quantitative manner the ability of the synthetic compound AF-615/30368009 (compound number 1 in Tables A and B, and Fig. 1) to inhibit the binding of Geminin to the protein miniCdt1 (which corresponds to the amino acid region 158-396 of the human Cdtl protein), by calculating the inhibition constant (KI).

Surface plasmon resonance spectroscopy (SPR) was performed at 25°C, using a Biosensor Biacore instrument (Biacore T100). About 6,000 RU of miniCdt1 protein were immobilized on a CMS Chip (Biacore) at pH 5.5, using amino coupling of Lysine residues. Concentrations series of the full-length Geminin and the synthetic chemical compound were simultaneously injected across the chip in a buffer containing 20 mM Hepes pH 7.5, 200 mM NaCl, 0.01% Tween 20 at a flow rate of 30µl/min. The concentrations used were 12, 36, 107, 322, 966nM for Geminin and 10 µM, 100µM, 1000µM for the compound. The injection of samples was carried out in the presence of 20% DMSO, to ensure high solubility.

As can be seen from the binding curves (Fig. 2), the synthetic chemical compound AF-615/30368009 at a concentration of 1000 µM exhibits inhibition of Geminin binding to miniCdt1. In the graphs of Fig. 2, the x axis represents the different concentrations of Geminin (nM), while the y-axis the binding levels in RU (Response Units). Based on the estimated value of the inhibition constant (KI), the synthetic compound AF-615/30368009 with KI = 0.75 ± 0.19mM (Fig. 2) displays a strong inhibitory effect in vitro. The results in Fig. 2 indicate the specificity of the in vitro inhibitory effect of the synthetic chemical compound AF-615/30368009 on the Geminin-miniCdt1 protein interaction.

### Example 2

**Ex vivo study of the ability of the synthetic compound AF-615/30368009 in disrupting and/or inhibiting formation of the Geminin-Cdtl protein complex**

A cellular assay was developed and applied using epithelial cells of breast adenocarcinoma (MCF7), for the purpose of the *ex vivo* study of the ability of the synthetic chemical compound AF-615/30368009 to inhibit formation and/or disrupt the exogenous Geminin-Cdtl protein complex.

The appropriate number of cells was seeded onto glass coverslips and cells were allowed in culture in order to adhere. Then they were transiently co-transfected with plasmid expression vectors pcDNA3.1-EGFP and pdiHcRed-N1 containing the DNA regions encoding for the proteins Cdtl-GFP and Geminin-dhcRed respectively. Three to five hours after co-transfection, the medium was removed and replaced with fresh medium which contained the synthetic chemical compound AF-615/30368009 at a concentration of 100µM.

After 24-hour incubation of the cells in the presence of the synthetic compound, the coverslips were washed with cold PBS 1X buffer (2 times), followed by fixation of the cells by incubation in 4% paraformaldehyde (PFA) for 10 minutes at room temperature. Next, coverslips were washed with 1x PBS buffer (2 times) and mounted on microscope slides using Mowiol. Mounting medium was allowed to dry for 16-18 hours at 4°C in the dark.

Culture of MCF7 cells was performed in a humidified chamber under conditions of 37°C and 5% CO₂ in DMEM medium (Gibco Cat.No.:41966-029) supplemented with 20% (v/v) fetal bovine serum (FBS, Gibco Cat.No.: 10106-169). The medium also contained the antibiotics penicillin and streptomycin (P/S, Gibco Cat.No.: 15140-122) at a final concentration of 1% (v/v).

MCF7 cancer cells fixed as described above were used to perform FRET acceptor photobleaching. In order to quantify the efficiency (E) of the FRET phenomenon, the following equation was used:
(4) E = (Dpost - Dpre)/Dpost, wherein Dpre and Dpost is the mean value of fluorescence intensity of the donor before and after photobleaching of the acceptor, respectively.

For the purposes of this invention the inhibitory effect of the synthetic compound AF-615/30368009 on the exogenous Geminin-Cdtl protein complex was calculated as the percent (%) change in efficiency (E) of the FRET phenomenon. Two transfection conditions were used as negative control samples. One condition related to the co-transfection of cells which were incubated with neither with the synthetic chemical compound nor with DMSO (control), and the second related to the co-transfection of cells that were incubated only with DMSO.

FRET acceptor photobleaching experiments were performed as described above, applying confocal laser microscopy. For this purpose, a Leica TCS SP5 system was used. The recording of images was performed with a 63x1.4 NA oil-immersion objective lens, at a scanning speed of 400Hz (in both directions), using 95.55µm diameter pinhole and image resolution at 512x512 pixels. Excitation of the donor (Cdt1-GFP) and the acceptor (Geminin-dhcRed) was performed with laser at a wavelength of 488nm and 561nm, respectively (5-7% of the maximum intensity). To bleach the acceptor (Geminin-dhcRed) repetitive bleaching was applied (three repeats of equal duration at 100% intensity of the laser at excitation wavelength of 561nm). The detection range of the emitted radiation of the donor (Cdt1-GFP) was set at 500-550nm. The choice of the above parameters was performed using the application FRET AB of the software Leica Microsystems LASAF.

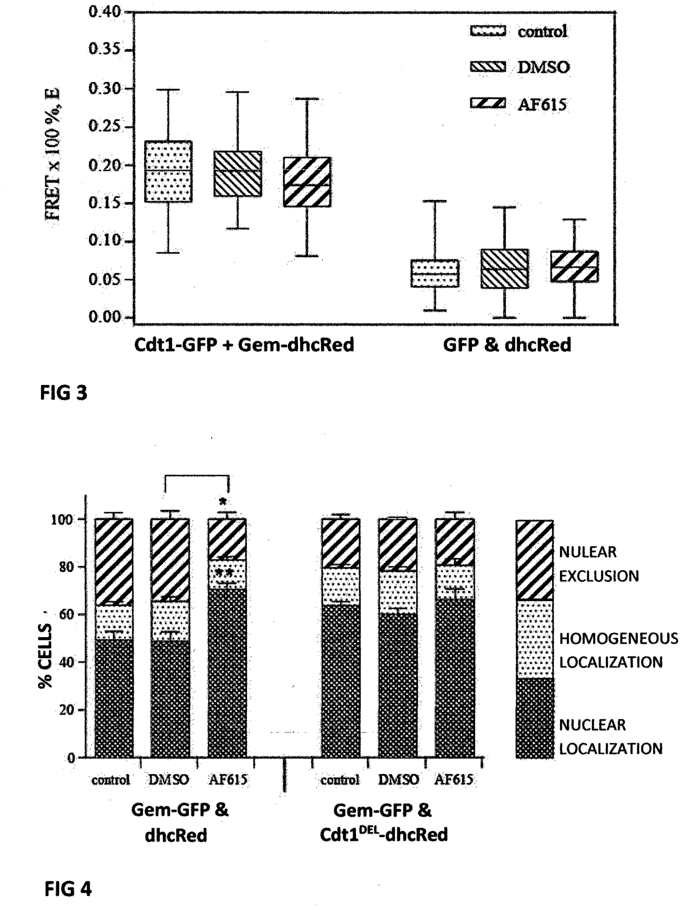

Fig. 3 shows the box plots for FRET efficiency values (E) per experimental condition, as indicated in the legend of the chart. The black continuous straight line within each box plot corresponds to the median of the total. For each box plot per experimental condition, the efficiency (E) of the FRET phenomenon was estimated in at least 40 positively co-transfected cells. Analysis of the results (Fig. 3) showed that the interaction between the proteins Cdt1-GFP & Geminin-dhcRed increased the (%) efficiency (E) of the FRET phenomenon, at least by three times compared to the background level, corresponding to the co-transfection with GFP & dhcRed. This result indicates strongly the specificity as well as the sensitivity of this cellular assay, in terms of the ex vivo detection and study of the Geminin-Cdtl protein interaction. Additionally, it indicates the suitability of said cellular assay for the ex vivo validation of the specificity of action of the synthetic compound AF-615/30368009.

A decrease in (E) suggests that a compound inhibits formation and/or disrupts the exogenous Geminin-Cdtl protein complex and hence is an indication of the specificity of its action *ex vivo.* Given that the population of MCF7 cancer cells was asynchronous, lower efficiency (E) due to inhibition of complex formation was expected in cells that are at the G1/S phase of the cell cycle, while correspondingly reduced efficiency (E) due to disruption of the complex was expected in cells that are in S, G2 and M phase of the cell cycle. The synthetic compound AF-615/30368009 (Fig. 3) caused a decrease in (E), although not statistically significant.

### Example 3

**Ex vivo study of the mechanism of action of the synthetic compound AF-615/30368009,** by **studying its effect on the intracellular localization of exogenous Geminin**

A cellular assay was developed and applied using epithelial cells of breast adenocarcinoma (MCF7), for the purpose of the *ex vivo* study of the mechanism by which the inhibitory effect of the synthetic chemical compound affects the functional significance of the Geminin-Cdtl protein interaction.

It is known that the nuclear localization of Geminin is necessary for the manifestation of its action concerning the regulation of DNA replication licensing (Yoshida K et al. Genes Cells. 2005, Vol. 10(1) pp 63-73). It has also been shown that Geminin-Cdtl interaction results in the translocation of Geminin to the nucleus, providing an additional spatio-temporal mechanism for Geminin regulation (Dimaki M et al. J Biol Chem. 2013, Vol. 288(33) pp 23953-63).

Therefore, in order to determine the mechanism of action of the synthetic chemical compound AF-615/30368009, its effect on the Geminin nucleo-cytoplasmic translocation was studied *ex vivo.*

For the purposes of this experiment, the full length human Geminin ortholog was used as well as the protein Cdt1^{DEL} which corresponds to the human ortholog which lacks the amino acid residues 170-190. Immunoprecipitation experiments have shown that the mutant protein Cdt1^{DEL} interacts weakly with Geminin, compared to the "wild-type" protein (Ballabeni A et al. EMBO J. 2004, Vol. 23(15) pp 3122-32).

For the implementation of the cellular assay, an appropriate number of cells were seeded onto glass coverslips and cells were allowed to culture in order to adhere. Next, they were transiently co-transfected with plasmid expression vectors pcDNA3.1-EGFP-N1 and pdiHcRed containing proteins Geminin-GFP and Cdt1^{DEL}-dhcRed respectively. Three to five hours after co-transfection, the medium was removed and replaced with fresh medium which contained the synthetic chemical compound AF-615/30368009 at a concentration of 100µM. After 24-hour incubation of the cells in the presence of the synthetic compound, the coverslips were washed with cold PBS 1X solution (2 times), followed by fixation of the cells by incubation in 4% PFA for 10 minutes at room temperature. Then, the coverslips were washed with 1x PBS buffer (2 times), mounted on microscope slides using mounting medium containing the dye DAPI (that stains only nuclei) and were observed under a fluorescence microscope.

The culture of MCF7 cells was performed in a humidified chamber under conditions of 37°C and 5% CO₂ in DMEM medium (Gibco Cat.No.: 41966-029) supplemented with 20% (v/v) FBS (Gibco Cat. No.: 10106-169). The medium also contained the antibiotics penicillin and streptomycin (P/S, Gibco Cat.No.: 15140-122) at a final concentration of 1% (v/v).

The experimental design included the transient co-transfection of MCF7 cancer cells with Geminin-GFP & dhcRed and Geminin-GFP & Cdt1^{DEL}-dhcRed. The co-transfection of Geminin-GFP & dhcRed was used as an internal control of the cellular assay, in order to determine whether it is possible to detect the change in intracellular localization of Geminin-GFP in the presence of Cdt1^{DEL}-dhcRed. The intracellular localization of Geminin was detected by the autofluorescence of the GFP molecule. Quantitation of autofluorescence data was performed by counting at least 100 double-positive transfected cells in different microscopic viewing areas, for each condition. The classification was based on the detection of the signal of the GFP molecule and included three groups: purely nuclear localization, purely cytoplasmic localization (nuclear exclusion) and homogeneous localization throughout the cell (nucleus and cytoplasm). The graph in Fig. 4 represents the averages of the measurements (expressed as %) for at least three independent experiments and the error bars represent the standard error of the means. The x-axis represents the two different conditions of transient co-transfection that were tested, while the y-axis the percentage (%) of co-transfected cells.

Cells that were co-transfected with Geminin-GFP & dhcRed showed higher percentage of nuclear exclusion of Geminin as compared to the cells that were co-transfected with Geminin-GFP & Cdt1^{DEL}-dhcRed (Fig. 4). This observation indicates the suitability and sensitivity of this cellular assay for the detection of the intracellular localization of Geminin-GFP.

To study the effects of the synthetic compounds on the intracellular localization of Geminin-GFP, co-transfected cells that were not incubated with the synthetic chemical compound or with DMSO, and cells that were incubated only with DMSO were used as controls. Based on the results (Fig. 4), the synthetic compound AF-615/30368009 caused a statistically significant increase in the percentage of cells with nuclear localization of Geminin, in the case of co-transfection with Geminin-GFP & dhcRed. One possible interpretation of this observation is that the mode of action of the compound mimics that of protein Cdtl, with regards to its ability to cause translocation of Geminin to the nucleus. In cells that were co-transfected with Geminin-GFP & Cdt1^{DEL}-dhcRed, the compound AF-615/30368009 caused no detectable change in intracellular localization of Geminin. This may be because the effect is not so strong as to effectively compensate for the presence of the exogenous protein Cdtl.

### Example 4

**Effect of the synthetic compound AF-615/30368009 in the cell cycle progression of cancer cells and normal cells**

Since the proteins Geminin and Cdtl are key regulators of the cell cycle, the effect of the synthetic compound on the development of the cancer cell and normal cell cycle was studied. Staining of the genetic material of the cells was performed with propidium iodide and the technique of flow cytometry was applied for their separation, based on the amount of genetic material.

MCF7 cells were used as cancer cells, whereas primary cells and particularly embryonic mouse fibroblasts (Mouse Embryonic Fibroblasts, MEFs) were used as normal cells. The cells were incubated with the synthetic chemical compound AF-615/30368009 at a concentration of 100 µM and cultured for 23hrs in 100-mm dishes, to 80% confluency. Cells which had been incubated with only DMSO were used as a negative control sample. The next day of the incubation, cells were harvested using trypsin. After a centrifugation step, the pellet was resuspended in 1X PBS and centrifuged at 800rpm for 5 minutes. Subsequently, cells were fixed in cold (4°C) 70% ethanol and stored for at least 16hrs at -20°C. After fixation, washes were performed with 1X PBS (2 times) and cells were finally resuspended in 1X PBS containing 2 µg/ml propidium iodide and 100 µg/ml RNaseA. The experiments were conducted using a Becton Dickinson flow cytometer and the data were analyzed by the WinMDi software version 2.8.

Cell culture of MEFs was performed in a humidified chamber under conditions of 37°C and 5% CO₂ in DMEM (Gibco) medium supplemented with 10% (v/v) FBS and 2 mM final concentration of glutamine (Gibco, Cat.No.:25030-024). The medium also contained the antibiotics penicillin and streptomycin (P/S, Gibco Cat.No.: 15140-122), at a final concentration of 1% (v/v).

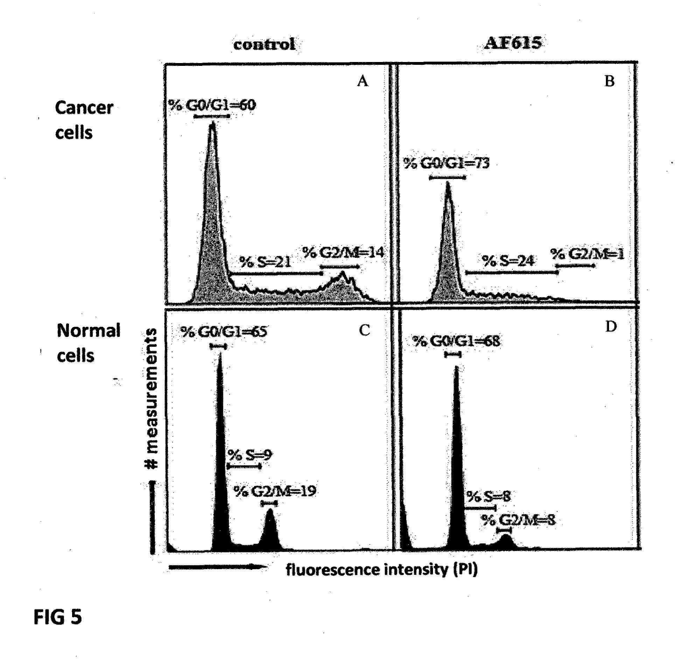

The graphs shown in Fig. 5 are representative of at least two independent experiments. The x axis represents the intensity of the fluorescence of propidium iodide and proportionally the amount of DNA content of the cells, while the y-axis corresponds to the number of events for each phase of the cell cycle.

The results (Fig. 5) showed that incubation of MCF7 cancer cells with the synthetic chemical compound AF-615/30368009 (Fig. 5B) caused a significant increase in the percentage (%) of cells in G0/G1 and S phases, compared to the control sample (Fig. 5A). Also, the population of cells corresponding to the G2/M phase of the cell cycle was extremely limited in comparison to the control (Fig. 5A). These results indicate that the synthetic compound AF-615/30368009 can lead to a cell cycle block at the G1/S transition in MCF7 cancer cells.

In order to determine the selectivity of action of the AF-615/30368009 synthetic chemical compound between cancer and normal cells, its effect on the cell cycle progression of primary cells (MEFs) was studied. The results showed that the compound AF-615/30368009 (Fig. 5D) caused no significant change in the percentage of cells in phase G0/G1 and S, and only a mild reduction to the corresponding percentage of cells in G2/M phase compared to the control sample (Fig. 5C). These results indicate that the compound AF-615/30368009 does not cause inhibition of cell cycle progression in normal cells, as pronounced as that observed in MCF7 cancer cells (Fig. 5B). This observation is of great importance, as it accounts for the selectivity and potency of this synthetic compound in cancer cells versus normal (primary) cells.

## Claims

1. A compound of the formula I for use in the treatment of cancer, wherein: 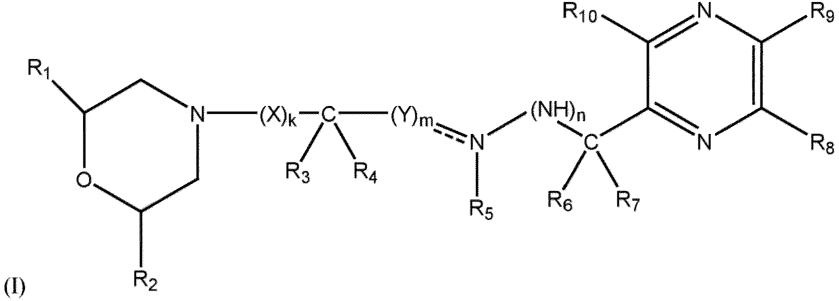
R₁ and R₂ are independently -H;
k is 0;
R₃ and R₄ together form =S ; R₆ and R₇ together form =O ;
m is 0;
R₅ is -H or CH₃;
n is 1;
R₈ is -H; R₉ is -H;
R₁₀ is -H;
or a stereoisomer, enantiomer and/or a pharmaceutically acceptable salt or hydrite thereof.

2. A compound selected among the group comprising N'-(morpholine-4-carbothioyl)pyrazine-2-carbohydrazide, N'-methyl-N'-(morpholine-4-carbothioyl)pyrazine-2-carbohydrazide, or a stereoisomer, enantiomer and/or a pharmaceutically acceptable salt or hydrite thereof, for use in the treatment of cancer.

3. A pharmaceutical composition which comprises a compound according to any one of claims 1 to 2, or a pharmaceutically acceptable salt thereof, in combination with one or more pharmaceutically acceptable carriers, diluents or excipients therefor, for use in the treatment of cancer.

## Patentansprüche

1. Eine Verbindung der Formel I zur Verwendung bei der Behandlung von Krebs, wobei: 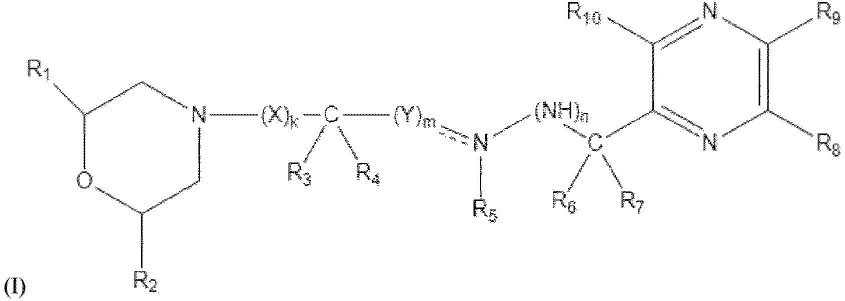
R₁ und R₂ sind unabhängig voneinander -H;
k ist 0;
R₃ und R₄ bilden zusammen =S; R₆ und R₇ bilden zusammen =O
m ist 0;
R₅ ist -H oder CH₃
n ist 1;
R₈ ist -H; R₉ ist -H;
R₁₀ ist -H;
oder ein Stereoisomer, Enantiomer und/oder ein pharmazeutisch verträgliches Salz oder Hydrit davon.

2. Zusammengestellt unter Auswahl der Gruppe, bestehend aus N'-(Morpholin-4-carbothioyl)pyrazin-2-carbohydrazid, N'-Methyl-N'-(Morpholin-4-carbothioyl)pyrazin-2-carbohydrazid oder einem Stereoisomer, Enantiomer und/oder einem pharmazeutisch verträglichen Salz oder Hydrit davon, zur Verwendung bei der Behandlung von Krebs.

3. Eine pharmazeutische Zusammensetzung, die eine Verbindung gemäß einem der Ansprüche 1 bis 2 oder ein pharmazeutisch verträgliches Salz davon in Kombination mit einem oder mehreren pharmazeutisch verträglichen Trägern, Verdünnungsmitteln oder Hilfsstoffen davon, zur Verwendung bei der Behandlung von Krebs.

## Revendications

1. Composé de formule I pour utilisation dans le traitement de cancer, dans laquelle: 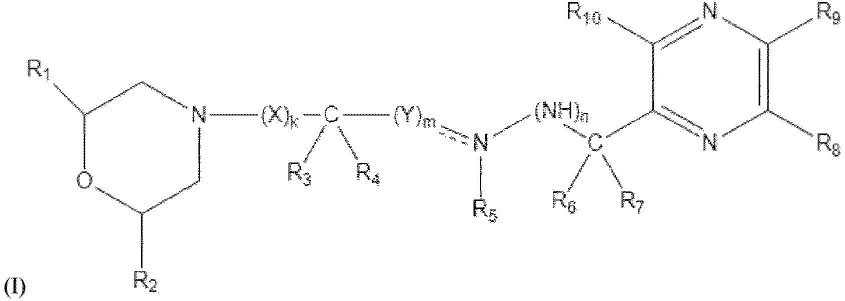
R₁ et R₂ sont indépendamment -H;
k est 0;
R₃ et R₄ forment ensemble =S; R₆ et R₇ forment ensemble =O
m est 0;
R₅ est -H ou CH₃
n est 1;
R₈ est -H; R₉ est -H;
R₁₀ est -H;
ou un stéréoisomère, un énantiomère et/ou un sel ou hydrite pharmaceutiquement acceptable de celui-ci.

2. Composé choisi parmi le groupe comprenant le N'-(morpholine-4-carbothioyl)pyrazine-2-carbohydrazide, le N'-methyl-N'-(morpholine-4-carbothioyl)pyrazine-2-carbohydrazide, ou un stéréoisomère, un énantiomère et/ou un sel ou hydrite pharmaceutiquement acceptable de celui-ci, pour utilisation dans le traitement de cancer.

3. Composition pharmaceutique qui comprend un composé selon l' une quelconque des revendications 1 à 2, ou un sel pharmaceutiquement acceptable de celui-ci, en combinaison avec un ou plusieurs véhicules, diluants ou excipients pharmaceutiquement acceptables de celui-ci, pour utilisation dans le traitement de cancer.
